# FASCICULE DE BREVET EUROPEEN

(11) **EP 2 157 969 B1**
(45) Date de publication et mention de la délivrance du brevet: **08.08.2012**
(21) Numéro de dépôt: 08806047.0
(22) Date de dépôt: 20.06.2008
(51) Int. Cl.: A61K 9/16

(54) **PROCEDE DE PREPARATION D'UNE FORME GRANULEE**
VERFAHREN ZUR HERSTELLUNG EINER GRANULIERTEN FORM
METHOD FOR PREPARING A GRANULATED FORM

(30) Priorité: 25.06.2007 FR 0755983
(43) Date de publication de la demande: 03.03.2010
(73) Titulaire: Laboratoires Aditec, 35800 Dinard (FR)
(72) Inventeur: COLLIN, Daniel, F-35400 Saint-Malo (FR)
(74) Mandataire: Hart-Davis, Jason
(86) Numéro de dépôt international: PCT/FR2008/051115
(87) Numéro de publication internationale: WO 2009/007573

(56) Documents cités:
- WO-A-2005/011737
- FR-A- 2 786 395

## Description

La présente invention a pour objet un procédé de préparation d'une forme granulée d'une composition comprenant au moins (A) un sucre, et (B) un composé comportant un groupe amino-NH₂,

Le procédé selon l'invention, comprenant l'utilisation d'un liant dans un solvant alcoolique et le séchage sous pression réduite, a l'avantage de permettre un stockage du produit granulé obtenu tout en évitant la réaction de Maillard.

### ETAT DE LA TECHNIQUE

La condensation de Maillard est le résultat de réactions se produisant entre les sucres réducteurs (oses) et les molécules possédant un groupement amino telles que les acides aminés (ainsi que les protéines et les peptides).

Elle conduit après isomérisation à la formation de glycosylamines N substituées qui subissent rapidement un réarrangement dit d'Amadori pour former des cétosamines.

Ces cétosamines, par scission, forment de petites molécules carbonylées qui par condensation aldolique donnent des polymères (mélanoïdes) caractéristiques de la réaction de Maillard. Le jaunissement ou brunissement dû à la réaction de Maillard peut être détecté par inspection visuelle. Le dosage de HMF (hydroxyméthylfurfural) permet également de suivre cette réaction.

Si la réaction de Maillard peut être bénéfique dans le domaine de l'industrie alimentaire, les modifications qui interviennent sur la couleur et la saveur des aliments pouvant être désirables, l'instabilité chimique des produits est bien entendu inacceptable dans le domaine pharmaceutique.

Il est constaté dans ce domaine que même des préparations pharmaceutiques, en principe sèches, montrent des signes de dégradation lors du stockage.

Un exemple de composition pharmaceutique dans laquelle le problème de la réaction de Maillard se présente se trouve dans le domaine des compositions réhydratantes permettant la réhydratation orale suivant une déshydratation résultant de troubles digestifs, tels que des diarrhées ou vomissements, ou comme conséquence d'une transpiration excessive due à la fièvre ou à la forte chaleur.

Les compositions réhydratantes utilisées dans ce contexte comprennent couramment à la fois des sucres et des acides aminés. Le problème d'éviter la réaction de Maillard se pose donc tout particulièrement dans ce domaine.

Une possibilité pour éviter les réactions de Maillard consiste naturellement dans la séparation physique des composés susceptibles de réagir. Par exemple, les compositions peuvent se présenter dans des emballages distincts, séparant sucres et acides aminés. Une autre possibilité, mais qui se révèle relativement coûteuse, consiste à enrober les acides aminés de manière à empêcher toute réaction avec le sucre, même si les deux sont présents dans une même composition solide, tel que décrit dans J-58-079962 ou JP-57-128662.

Une autre possibilité pour éviter la réaction de Maillard consiste à rajouter un composé supplémentaire qui agit comme inhibiteur compétitif en réagissant à la place d'au moins un des membres du couple principal sucre-amine au coeur de la composition. Une telle solution est proposée dans le brevet US 4 371 557.

Encore une possibilité connue dans ce domaine est celle illustrée par les demandes de brevet WO 00/32170 et FR 2 786 395. Ces documents décrivent des compositions solides contenant des amines et des sucres, dans lesquels les groupes amino sont protégés par des groupes protecteurs. Il s'agit notamment de dérivés carbonates d'acides aminés, tels que le carbonate de glycine ou le bicarbonate d'arginine. C'est au moyen de la protection des groupes amino que l'on empêche la réaction de Maillard, les groupes carbonates étant éliminés en présence d'eau lors de l'utilisation finale des compositions décrites.

### DESCRIPTION DE L'INVENTION

Il a maintenant été découvert, de manière tout à fait surprenante, qu'un procédé de granulation dans un solvant alcoolique sous pression réduite et à basse température permet d'obtenir des formes granulées comprenant un mélange intime à la fois de sucre et de composés comprenant un groupe amino et que lesdites formes granulées sont stables dans le temps.

Ainsi, la présente invention porte sur un procédé de préparation d'une forme granulée d'une composition comprenant au moins (A) un sucre et (B) un composé comportant un groupe amino -NH₂, ledit procédé comprenant au moins les étapes de :
(a) une ou plusieurs étape(s) de prémélange de constituants secs comprenant au moins (A) un sucre et (B) un composé comportant un groupe amino -NH₂;
(b) une ou plusieurs étape(s) de mouillage du prémélange de constituants secs obtenu à l'étape (a) au moyen d'une solution de liant (C) dans un solvant alcoolique (D) ;
(c) une ou plusieurs étape(s) de granulation du mélange mouillé obtenu à l'étape (b) ;
(d) une ou plusieurs étape(s) de séchage sous pression réduite à une température ne dépassant pas 47°C.

Ce procédé est particulièrement avantageux pour la préparation de compositions pharmaceutiques. Ainsi, le procédé de la présente invention trouvera notamment application dans la préparation de compositions réhydratantes, D'autres compositions à préparer dans le domaine pharmaceutique ainsi que dans le domaine des compléments alimentaires peuvent nécessiter l'association d'un sucre et d'un acide aminé, et le procédé de la présente invention peut donc y trouver application.

A ce titre, les sucres et saccharides couramment utilisés dans de telles compositions constituent un mode de réalisation préférentiel de la présente invention.

Le sucre (A) sera donc choisi avantageusement dans le groupe constitué par des monosaccharides, disaccharides et polysaccharides. Plus particulièrement, le sucre (A) sera choisi avantageusement parmi les hexoses tels que le galactose, le glucose, le fructose et le mannose ; les pentoses tels que l'arabinose, le xylose et le ribose ; les diholosides non réducteurs tels que le saccharose ou le tréhalose ou réducteurs tels que le lactose, le maltose, la cellobiose ou l'isomaltose ; les triholosides tels que le raffinose ou le gentianose. Le lactulose peut également constituer un composant de la partie sucre (A) de la composition à préparer. Un polyholoside tel que l'amidon et la cellulose peut également jouer le rôle de sucre (A) dans le procédé de la présente invention. Un seul sucre ou une combinaison de sucres peut constituer le composant sucre (A) de la présente invention.

De préférence, au moins une partie du composant sucre (A) sera constituée de lactose, préférentiellement du lactose sous forme cristalline alpha monohydratée. Une quantité minimale préférée de lactose sera généralement de l'ordre de 15% en poids de lactose par rapport au poids total du produit final granulé pris comme 100%.

Dans des variantes préférées, le procédé de l'invention utilisera des compositions comprenant, en tant que composant (A), à la fois du lactose et un autre sucre choisi parmi : le dextrose ; le fructose ; le saccharose ; le lactulose. Il est également possible de combiner le lactose avec des sucres alcools tels que le xylitol ou le sorbitol.

De manière analogue, des composés aminés d'un intérêt particulier dans la présente invention seront les acides aminés couramment utilisés dans les compositions réhydratantes. Ainsi, le composé comportant un groupe amino-NH₂ sera avantageusement au moins un choisi dans le groupe constitué par : la lysine, l'arginine, la proline, l'alanine, l'asparagine, l'acide aspartique, la cystéine, l'acide glutamique, la glutamine, la glycine, l'histidine, l'isoleucine, la leucine, la méthionine, la phénylalanine, la sérine, la thréonine, le tryptophane, la tyrosine et la valine.

La glycine, la méthionine, la lysine, le tryptophane, la glutamine constituent un groupe d'acides aminés présentant un intérêt particulier pour la préparation de compositions réhydratantes et d'autres compositions selon le procédé de la présente invention.

La granulation selon la présente invention nécessite la présence d'un liant en solution alcoolique.

Le liant (C) est un composé organique pouvant relier des particules des matières solides entre elles pour former des grains. On utilise un polymère organique soluble dans des solvants alcooliques, mais acceptable pour des utilisations alimentaires et pharmaceutiques. Il a été trouvé que la polyvinylpyrrolidone permet d'agir efficacement comme liant dans le procédé de la présente invention et celle-ci constitue donc un liant préféré. Les gommes de type acacia, xanthane, caroube et carraghénanes peuvent également être utilisées en tant que liant dans le procédé de la présente invention. Un ou plusieurs liants peuvent être combinés pour former le composant liant (C).

Le solvant alcoolique (D) de la présente invention doit être suffisamment volatil pour permettre son enlèvement lors de l'étape (d) de séchage. Ainsi l'utilisation de alcools en C₄ et plus n'est pas préférée dans le procédé de la présente invention. Le méthanol présente une toxicité élevée est de ce fait n'est pas préféré. Les alcools à utiliser seront avantageusement choisis parmi : l'éthanol, l'alcool propylique et l'alcool isopropylique, plus préférentiellement l'alcool isopropylique. On choisira un grade d'alcool présentant une quantité minimale d'eau, de préférence inférieure à 0,1 % (en poids) et plus préférentiellement inférieure à 0,01 %.

Selon un mode préférentiel de réalisation de la présente invention, la composition à préparer comprend un couple effervescent. De préférence, ce couple effervescent est constitué par le mélange d'un (hydrogéno)carbonate alcalin et d'un acide organique ou minéral, le (hydrogéno)carbonate alcalin étant choisi parmi le carbonate de calcium, le carbonate de sodium, le carbonate de potassium, l'hydrogénocarbonate de sodium, l'hydrogénocarbonate de potassium ainsi que des mélanges de ces composés, et l'acide organique ou minéral étant choisi parmi l'acide citrique, l'acide fumarique, l'acide adipique, l'acide tartrique, le phosphate monosodique et le phosphate monopotassique ainsi que des mélanges de ces composés.

Il a été remarqué que le procédé de l'invention permet non seulement d'inhiber la réaction de Maillard mais également d'empêcher le déclenchement d'une réaction effervescente.

Au moins certaines des étapes (a), (b), (c) et (d) peuvent être en réalité constituées de deux ou plusieurs sous-étapes. Ainsi les étapes (b) mouillage, (c) granulation et (d) séchage peuvent avantageusement être constituées d'au moins deux étapes avec des conditions opératoires légèrement différentes.

Selon un mode préférentiel de réalisation, un cycle de prémélange permet d'homogénéiser l'ensemble des matières premières. La vitesse des mobiles d'agitation mécaniques du réacteur de granulation, telle que la pale et le couteau ainsi que sa profondeur (hauteur du couteau dans le réacteur) permettent d'optimiser l'homogénéité du lot. L'ajout des matières premières préalablement à l'étape (a) de prémélange peut avoir lieu par chargement direct du réacteur de granulation sans différence de pression, ou peut comprendre l'utilisation du vide pour aspirer les matières premières solides vers l'intérieur du réacteur. Dans les deux cas, les matières premières solides étant à l'air libre, et le chargement impliquant l'incorporation d'une certaine quantité d'air ambiant, il est préférable de régler l'humidité relative de l'air dans la salle où se situe le granulateur à une valeur comprise entre 15 et 40%, et de préférence entre 15 et 30%. De même, il est préférable de régler la température de l'air dans la salle où se situe le granulateur à une valeur comprise entre 15 et 30°C, et de préférence entre 15 et 25°C.

Encore selon un mode préférentiel de réalisation, deux cycles de mouillage avec des vitesses de pale différentes permettent un mélange intime du liant avec les poudres, et deux cycles de granulation avec des vitesses de pale différentes et un réglage du couteau en vitesse et en profondeur provoquent la granulation du mélange. L'énergie mécanique fournie au stade du mouillage est relativement faible et peut avantageusement être apportée au moyen de la pale seule. En revanche, au stade de la granulation, une quantité plus importante d'énergie mécanique est apportée au mélange, afin de provoquer la granulation. Cette énergie supplémentaire peut avantageusement être apportée en faisant appel au(x) couteau(x) du réacteur en plus d'une pale au fond du réacteur. Une telle mise en oeuvre permet d'obtenir un ensemble de particules de taille relativement homogène.

Encore selon un mode préférentiel de réalisation il y aura deux cycles de séchage : un premier cycle où la mise sous vide provoque l'évaporation du solvant et le refroidissement du produit ; un second cycle avec chauffage par la double enveloppe de la cuve (la double enveloppe peut contenir un fluide circulant à température variable) permet de finaliser le séchage.

Le réacteur granulateur est soumis lors du séchage à un mouvement d'oscillation, ce qui maintient le produit en contact de la paroi chauffante sans provoquer de surchauffe du produit. L'agitation est maintenue par la pale de manière à ce que les granulés viennent au contact de la paroi chaude, de façon uniforme, avec une périodicité constante pour éviter la surchauffe du produit, ce qui provoquerait la réaction de Maillard. Le vide permet d'éliminer les inconvénients d'un séchage à l'air chaud, l'humidité (eau libre et eau de constitution) des granulés étant évaporée à une température plus basse, ce qui permet d'obtenir un séchage plus rapide et de conserver le produit à une température suffisamment basse pour ne pas déclencher de réaction de Maillard.

Il peut être avantageux après l'étape (ou les étapes) de séchage de refroidir le produit afin de le stabiliser. L'eau froide dans la double enveloppe du réacteur est utilisé pour faire descendre la température des grains séchés jusqu'à la température ambiante. Le vide est utilisé pour vider le granulateur, afin de conserver une humidité relative très basse du milieu.

Après la production et séchage des granules, il peut être avantageux de classifier les granules obtenues par un procédé de tamisage. Comme il a été indiqué plus haut pour le rajout des matières premières solides, le tamisage étant le plus souvent et de manière pratique effectué à l'air libre, on préférera ajuster les conditions climatiques dans la salle de tamisage, en ajustant l'humidité relative à une valeur comprise entre 15 et 40%, et de préférence entre 15 et 30%, et en ajustant la température de l'air à une valeur comprise entre 15 et 30°C, et de préférence entre 15 et 25°C.

Selon un mode de réalisation préférentiel de la présente invention, le procédé selon l'invention est réalisé dans un réacteur présentant les caractéristiques suivantes :
- fermeture hermétique permettant le maintien d'une pression réduite et moyens d'assurer un vide partiel;
- buse(s) permettant d'ajouter une solution liante
- cuve basculante permettant l'oscillation lors du séchage ;
- parois à température variable pouvant être chauffées de manière à induire le séchage des grains ;
- une pale au fond du réacteur ;
- au moins un couteau à longueur variable fixé au couvercle du réacteur ;
- une vanne de déchargement pour le retrait du produit.

Des réacteurs de ce type sont commercialisés par la société ZANCHETTA sous la marque ROTO P.

De manière préférentielle, la pression réduite pendant l'étape de séchage (d) sera inférieure à 50 mbars.

De manière préférentielle, le procédé sera conduit de manière à préparer une forme granulée présentant une valeur résiduelle d'eau telle que mesurée par la méthode Karl Fischer qui soit inférieure à 0,9 % et/ou une valeur aw (*activity of water,* activité de l'eau) inférieure à 0,2. La valeur aw est un rapport sans dimensions entre la pression de vapeur de l'eau d'un produit et la pression de vapeur de l'eau de l'eau pure à la même température, et permet de déterminer l'eau « libre », non liée par la matrice d'un produit alimentaire ou pharmaceutique et ainsi disponible pour les réactions chimiques ou microbiologiques qui pourraient conduire à la dégradation du produit.

Il a été constaté que les produits sous forme granulée préparés selon la présente invention ne montrent pas les signes caractéristiques de la réaction de Maillard, tels que le jaunissement ou brunissement. De plus, on ne constate pas de perte d'intensité d'effervescence au cours du temps, ni de déliquescence du produit granulé.

La stabilité des formes solides est remarquable en ce sens qu'elle perdure pendant de longues périodes de stockage sans précautions particulières pour exclure l'humidité (sauf conditionnement pharmaceutique normal) ni pour stocker à une température en dessous de la température ambiante. Cette stabilité est d'un intérêt particulier au vu de la tendance notoire des compositions associant sucres et amines à se dégrader au cours du temps, même à l'état solide.

### Exemples

### Exemple 1

Un exemple de protocole de fabrication actuellement préféré, et notamment pour un réhydratant granulé effervescent, se constitue en huit cycles :

### 1^{er} cycle : prémélange

- Prémélange des matières, vitesse de la pale à 75 tr/mn
- Vitesse du couteau (chopper) : 700 tr/mn, profondeur du couteau : 80 %
- Durée du cycle : 5 minutes

### 2^{ème} cycle : mouillage 1

- Pulvérisation de la solution liante (alcool isopropylique + kollidon K30) à un débit de 14 l/h
- Vitesse de la pale à 60 tr/mn
- Durée du cycle : 120 s

### 3^{ème} cycle: mouillage 2

- Pulvérisation de la solution liante (alcool isopropylique + kollidon K30) à un débit de 14 l/h
- Vitesse de la pale à 100 tr/mn
- Durée du cycle : 700 s

### 4^{ème} cycle : granulation 1

- Vitesse de la pale pour la granulation du mélange à 100 tr/mn
- Vitesse du couteau (chopper) : 700 tr/mn, profondeur : 65 %
- Durée du cycle : 5 mn

### 5^{ème} cycle : granulation 2

- Vitesse de la pale pour la granulation du mélange à 80 tr/mn
- Vitesse du couteau (chopper) : 700 tr/mn, profondeur : 65 %
- Durée du cycle : 5 mn

### 6^{ème} cycle : séchage 1

- Séchage sous vide du mélange sans monter en température avec une pression comprise entre 10 et 50 mbars et de préférence 10 mbars
- Vitesse de la pale à 5 tr/mn avec des séquences de fonctionnement et d'arrêt de 120 s
- Vitesse du couteau (chopper) : 700 tr/mn, profondeur : 100%
- Durée du cycle : 600 s

### 7^{ème} cycle : séchage 2

- Séchage sous vide avec oscillation du granulateur à 170°C, avec une pression comprise entre 10 et 50 mbars et de préférence 10 mbars, température de la double enveloppe : 55°C
- Vitesse de la pale à 5 tr/mn avec des séquences de fonctionnement et d'arrêt de 120 s
- Vitesse du couteau (chopper) : 700 tr/mn, profondeur : 100%
- Durée du cycle : 10500 s
- La température maximum du produit ne dépasse pas 47°C
- Le taux d'humidité mesuré par la méthode de Karl Fisher est compris entre 0 et 0.9 % et de préférence ≤ 0.2 %

### 8^{ème} cycle : refroidissement

- Refroidissement sous vide avec une pression comprise entre 10 et 50 mbars et de préférence 10 mbars, température de la double enveloppe : 10°C
- Vitesse de la pale à 5 tr/mn avec des séquences de fonctionnement de 60 s et d'arrêt de 180 s
- Le couteau (chopper) est à l'arrêt
- Durée du cycle : 2400 s

Les matières premières sèches sont combinées dans un granulateur présentant les dimensions suivantes : hauteur du fond jusqu'à la coupole : 800 mm, diamètre de la cuve 1200 mm, diamètre de la pale 1100 mm. Le « chopper » est constitué d'un système de trois couteaux (tous de forme trapézoïdale) séparés au moyen de deux anneaux. Le premier couteau de forme trapézoïdale présente une longueur inférieure de 90 mm, une longueur supérieure de 200 mm, une largeur de 30 mm et une épaisseur de 6 mm. Le deuxième couteau de forme trapézoïdaie présente les mêmes dimensions que le premier couteau. Le troisième couteau présente une longueur inférieure de 70 mm, une longueur supérieure de 240 mm, une largeur de 95 mm et une épaisseur de 8 mm. Les deux anneaux sont de diamètre 50 mm et d'épaisseur 10 mm.

La solution de liant introduite lors des étapes de mouillage est préparée par mélange (dans une cuve de 100 litres) de 20 kg de Kollidon® K30 commercialisée par la société BASF (une préparation de polyvinylpyrrolidone) et de 47,5 kg d'alcool isopropylique.

Les grains obtenus par le procédé sont criblés par un tamis de 2 mm, les grains de taille supérieure étant renvoyés pour être broyés à nouveau.

Le protocole décrit ci-dessus a été utilisé dans la préparation d'une composition réhydratante effervescente, qui se constitue comme suit :

### FORMULATION

| | | | |
|---|---|---|---|
| Bicarbonate de sodium | 13,51 % | 54,04 kg | anhydre |
| Acide citrique | 7,72 % | 30,88 kg | anhydre |
| Lactose 200 M | 64,21 % | 256,84 kg | Mono hydraté |
| Chlorure de sodium | 3,51 % | 14,04 kg | anhydre |
| Glycine | 4,53 % | 18,12 kg | anhydre |
| Chlorure de potassium | 1,49 % | 5,96 kg | anhydre |
| Ethyl vanilline | 0,03 % | 0,12 kg | anhydre |
| Kollidon K30 | 5,00 % | 20,00 kq | anhydre |
| | 100,00 % | 400,00 kg | |

### Exemple 2

Dans un deuxième exemple de réalisation du procédé selon la présente invention, une formulation contenant les composants suivants a été préparée :

| | |
|---|---|
| Bicarbonate de sodium | 31,070 kg |
| Acide citrique | 31,070 kg |
| Carbonate de sodium | 6,901 kg |
| Lactose 200 Mesh | 208,500 kg |
| Ethyl Vanilline | 1,125 kg |
| Choline Chlorure 50% SiO₂ | 1,500 kg |
| Lysine | 0,758 kg |
| Methionine | 0,758 kg |
| Tryptophane | 0,038 kg |
| Vitamine A | 0,375 kg |
| Vitamine D3 | 0,047 kg |
| Vitamine C | 0,750 kg |
| Vitamine E | 0,750 kg |
| Vitamine K3 | 0,150 kg |
| Vitamine B3 | 0,474 kg |
| Vitamine B12 | 0,250 kg |
| Vitamine B2 | 0,117 kg |
| Vitamine B5 | 0,128 kg |
| Vitamine B1 | 0,077 kg |
| Vitamine B6 | 0,048 kg |
| Vitamine B9 | 0,020 kg |
| Vitamine H | 0,094 kg |
| Kollidon K30 | 7,500 kg |
| Cernivet LBC ME 10 | 7,500 kg |

La solution de liant utilisée dans ce deuxième exemple était préparée par agitation pendant une durée de 1 heure dans une cuve de 100 L de 7,5 kg de Kollidon^{®} K30 et de 37,5 kg d'alcool isopropylique.

Le procédé utilisé se constituait de huit étapes et était analogue à celui décrit dans l'Exemple 1, exception faite des différences suivantes : durée du 3^{ième} cycle (mouillage 2): 280 s ; durée du 4^{ième} cycle (granulation 1): 120 s ; durée du 5^{ième} cycle (granulation 2): 440 s, avec vitesse de la pale : 160 tr/min ; durée du 7^{ième} cycle (séchage 2): 16800 s. De plus, dans ce deuxième exemple, le 8^{ième} cycle (refroidissement) a été effectué sous vide avec oscillation du granulateur à 170°.

### Test de stabilité après mise en sachet : observations

A l'issue de la fabrication selon le procédé de l'invention, la poudre granulée obtenue selon l'Exemple 1 ci-dessus a été conditionnée à une humidité relative de 30 % en sachets de 5 g. Les sachets ont été conservés à température ambiante (25°C) et ont été observés régulièrement et comparés à un témoin obtenu par simple mélange des constituants et conditionnés dans les mêmes sachets dans les mêmes conditions d'hygrométrie.

Au bout de quelques jours, on a noté sur le témoin une légère prise en masse et un début de formation de croûte du produit en surface. Le témoin a perdu de son effervescence (temps de dissolution augmenté de 200%). Au bout de deux semaines, le produit témoin ne se solubilisait plus. On a constaté une absence d'effervescence résiduelle. Le produit était de couleur brune. En revanche, la forme granulée préparée selon l'invention n'a pas montré au bout de six mois de modification significative de la couleur blanc cassé. Aucune prise en masse n'a été détectée ni perte d'intensité d'effervescence (le temps de dissolution était stable à 45 s).

De manière analogue, à l'issue de la fabrication selon le procédé de l'invention, la poudre granulée obtenue selon l'Exemple 2 ci-dessus a été conditionnée à une humidité relative de 30 % en sachets de 5 g. Les sachets ont été conservés à deux températures (ambiante (25°C) et 40°C) et ont été observés régulièrement et comparés à un témoin obtenu par simple mélange des constituants et conditionnés dans les mêmes sachets dans les mêmes conditions d'hygrométrie.

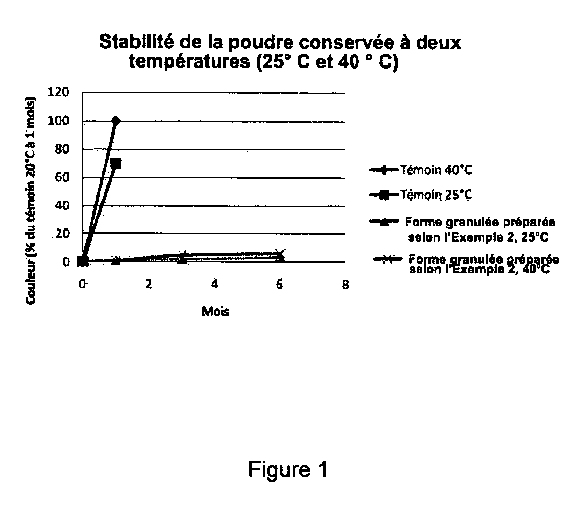

La Figure 1 matérialise la variation de couleur du produit granulé préparé selon l'Exemple 2 par rapport au produit témoin, étudiée aux températures de 25°C et de 40°C. A une température de 40°C, le produit témoin présente au bout d'un mois une couleur brune intense.

## Revendications

1. Procédé de préparation d'une forme granulée d'une composition comprenant au moins (A) un sucre et (B) un composé comportant un groupe amino -NH₂, ledit procédé comprenant au moins les étapes de :
(a) une ou plusieurs étape(s) de prémélange de constituants secs comprenant au moins (A) un sucre et (B) un composé comportant un groupe amino -NH₂;
(b) une ou plusieurs étape(s) de mouillage du prémélange de constituants secs obtenu à l'étape (a) au moyen d'une solution de liant (C) dans un solvant alcoolique (D) ;
(c) une ou plusieurs étape(s) de granulation du mélange mouillé obtenu à l'étape (b) ;
(d) une ou plusieurs étape(s) de séchage sous pression réduite à une température ne dépassant pas 47°C.

2. Procédé selon la revendication 1 dans lequel le sucre (A) est choisi dans le groupe constitué par : le galactose, le glucose, le fructose, le mannose, l'arabinose, le xylose, le ribose, le saccharose, le tréhalose, le lactose, le maltose, la cellobiose, l'isomaltose, le raffinose, le gentianose, le lactulose.

3. Procédé selon la revendication 1 ou 2 dans lequel la partie (A) sucre comprend au moins 15% en poids de lactose par rapport au poids total de la composition solide sous forme granulée à préparer.

4. Procédé selon la revendication 3, dans lequel outre le lactose, la partie (A) sucre comprend un sucre choisi parmi: le dextrose, le fructose, le saccharose et/ou le lactulose, et/ou comprend au moins un sucre alcool choisi parmi le xylitol et le sorbitol.

5. Procédé selon l'une quelconque des revendications 1 à 4 dans lequel le composé (B) comportant un groupe amino -NH₂ est un acide aminé.

6. Procédé selon la revendication 5 dans lequel l'acide aminé est choisi dans le groupe constitué par : la glycine, la méthionine, la glutamine, la lysine, le tryptophane.

7. Procédé selon l'une quelconque des revendications 1 à 6 dans lequel la composition à préparer comprenant au moins (A) un sucre et (B) un composé comportant un groupe amino -NH₂, est une composition réhydratante.

8. Procédé selon l'une quelconque des revendications 1 à 7 dans lequel le liant (C) est constitué par au moins choisi parmi : la polyvinylpyrrolidone, les gommes de type acacia, xanthane, caroube et carraghénanes.

9. Procédé selon l'une quelconque des revendications 1 à 8 dans lequel le solvant alcoolique (D) est au moins un choisi dans le groupe constitué par ; l'éthanol, l'alcool propylique et l'alcool isopropylique.

10. Procédé selon l'une quelconque des revendications 1 à 9 dans lequel la composition comprend en outre un couple effervescent.

11. Procédé selon la revendication 10 dans lequel le couple effervescent est constitué par le mélange d'un (hydrogéno)carbonate alcalin et d'un acide organique ou minéral, le (hydrogéno)carbonate alcalin étant choisi parmi le carbonate de calcium, le carbonate de sodium, le carbonate de potassium, l'hydrogénocarbonate de sodium, l'hydrogénocarbonate de potassium ainsi que des mélanges de ces composés, et l'acide organique ou minéral étant choisi parmi l'acide citrique, l'acide fumarique, l'acide adipique, l'acide tartrique, le phosphate monosodique et le phosphate monopotassique ainsi que des mélanges de ces composés.

12. Procédé selon l'une quelconque des revendications 1 à 11 dans lequel on effectue au moins une étape de séchage (d) sous pression réduite à une pression inférieure à 50 mbars.

13. Procédé selon l'une quelconque des revendications 1 à 12 dans lequel les étapes (a) à (d) sont réalisées dans un réacteur présentant les caractéristiques suivantes :
- fermeture hermétique permettant le maintien d'une pression réduite et moyens d'assurer un vide partiel;
- buse(s) permettant d'ajouter une solution liante
- cuve basculante permettant l'oscillation lors du séchage ;
- parois à température variable pouvant être chauffées de manière à induire le séchage des grains ;
- une pale au fond du réacteur ;
- au moins un couteau à longueur variable fixé au couvercle du réacteur ;
- une vanne de déchargement pour le retrait du produit.

## Claims

1. A method for preparing a granulated form of a composition comprising at least (A) one sugar and (B) a compound including an amino acid group -NH₂, said method comprising at least the steps of:
(a) one or several steps for pre-mixing dry constituents comprising at least (A) one sugar and (B) a compound including an amino acid group -NH₂;
(b) one or several steps for wetting the premix of dry constituents obtained in step (a) by means of a binder solution (C) in an alcoholic solvent (D);
(c) one or several steps for granulating the wet mixture obtained in step (b);
(d) one or several steps for drying under reduced pressure at a temperature not exceeding 47°C.

2. The method according to claim 1, wherein the sugar (A) is selected from the group formed by: galactose, glucose, fructose, mannose, arabinose, xylose, ribose, saccharose, trehalose, lactose, maltose, cellobiose" isomaltose, raffinose, gentianose, lactulose.

3. The method according to claim 1 or 2, wherein the sugar portion (A) comprises at least 15% by weight of lactose, based on the total weight of the solid composition in granulated form to be prepared.

4. The method according to claim 3, wherein in addition to lactose, the sugar portion (A) comprises a sugar selected from: dextrose, fructose, saccharose and/or lactulose, and/or comprises at least one sugar alcohol selected from xylitol and sorbitol.

5. The method according to any of claims 1 to 4, wherein the compound (B) including an amino acid group -NH₂ is an amino acid.

6. The method according to claim 5, wherein the amino acid is selected from the group formed by: glycine, methionine, glutamine, lysine, tryptophan.

7. The method according to any of claims 1 to 6, wherein the composition to be prepared comprising at least (A) one sugar and (B) a compound including an amino acid group -NH₂, is a rehydrating composition.

8. The method according to any of claims 1 to 7, wherein the binder (C) is formed by, at least selected from: polyvinylpyrrolidone, gums of the acacia, xanthan, carob type and carrageenan gums.

9. The method according to any of claims 1 to 8, wherein the alcoholic solvent (D) is at least one selected from the group formed by: ethanol, propyl alcohol and isopropyl alcohol.

10. The method according to any of claims 1 to 9, wherein the composition further comprises an effervescent pair.

11. The method according to claim 10, wherein the effervescent pair is formed by the mixture of an alkaline (hydrogen) carbonate and of an organic or mineral acid, the alkaline (hydrogen) carbonate being selected from calcium carbonate, sodium carbonate, potassium carbonate, sodium hydrogen carbonate, potassium hydrogen carbonate as well as mixtures of these compounds, and the organic or mineral acid being selected from citric acid, fumaric acid, adipic acid, tartaric acid, monosodium phosphate and monopotassium phosphate as well as mixtures of these compounds.

12. The method according to any of claims 1 to 11, wherein at least one drying step (d) is carried out under reduced pressure, at a pressure of less than 50 mbars.

13. The method according to any of claims 1 to 12, wherein steps (a) to (d) are carried out in a reactor having the following characteristics:
- a hermetic seal allowing a reduced pressure to be maintained and means for ensuring a partial vacuum;
- nozzle(s) allowing addition of a binding solution
- pivoting tank allowing oscillation during drying;
- walls with a variable temperature which may be heated so as to induce drying of the grains;
- a vane at the bottom of the reactor;
- at least one knife with a variable length attached to the lid of the reactor;
- an unloading valve for withdrawing the product.

## Patentansprüche

1. Verfahren zur Herstellung einer granulierten Form einer Zusammensetzung, die wenigstens (A) einen Zucker und (B) eine Verbindung mit einer Aminoguppe -NH₂ umfaßt, wobei das Verfahren wenigstens die Schritte umfaßt,:
(a) einen oder mehrere Schritt(e) zum Vormischen von trockenen Bestandteilen, die wenigstens (A) einen Zucker und (B) eine Verbindung mit einer Aminogruppe -NH₂ umfassen,
(b) einen oder mehrere Schritt(e) zum Befeuchten der bei Schritt (a) erhaltenen Vormischung aus trockenen Bestandteilen mittels einer Bindemittellösung (C) in einem alkoholischen Lösungsmittel (D),
(c) einen oder mehrere Schritt(e) zum Granulieren der bei Schritt (b) erhaltenen befeuchteten Mischung,
(d) einen oder mehrere Schritt(e) zum Trocknen unter vermindertem Druck bei einer Temperatur, die 47 °C nicht überschreitet.

2. Verfahren nach Anspruch 1, wobei der Zucker (A) aus der Gruppe bestehend aus Galaktose, Glukose, Fruktose, Mannose, Arabinose, Xylose, Ribose, Saccharose, Trehalose, Laktose, Maltose, Cellobiose, Isomaltose, Raffinose, Gentianose, Lactulose ausgewählt ist.

3. Verfahren nach Anspruch 1 oder 2, wobei der Zucker-Teil (A) wenigstens 15 Gew.-% Laktose bezogen auf das Gesamtgewicht der herzustellenden festen Zusammensetzung in granulierter Form umfaßt.

4. Verfahren nach Anspruch 3, wobei der Zucker-Teil (A) außer Laktose einen Zucker umfaßt, der aus Dextrose, Fruktose, Saccharose und/oder Lactulose ausgewählt ist, und/oder wenigstens einen Zuckeralkohol ausgewählt aus Xylit und Sorbit umfaßt.

5. Verfahren nach einem der Ansprüche 1 bis 4, wobei die eine Aminoguppe -NH₂ umfassende Verbindung (B) eine Aminosäure ist.

6. Verfahren nach Anspruch 5, wobei die Aminosäure aus der Gruppe bestehend aus Glycin, Methionin, Glutamin, Lysin, Tryptophan ausgewählt ist.

7. Verfahren nach einem der Ansprüche 1 bis 6, wobei die herzustellende Zusammensetzung, die wenigstens (A) einen Zucker und (B) eine Verbindung mit einer Aminoguppe -NH₂ umfaßt, eine rehydratisierende Zusammensetzung ist.

8. Verfahren nach einem der Ansprüche 1 bis 7, wobei das Bindemittel (C) von wenigstens, ausgewählt aus Polyvinylpyrrolidon, Gummis vom Typ Arabicum, Xanthan, Carubin und Carrageen gebildet ist.

9. Verfahren nach einem der Ansprüche 1 bis 8, wobei das alkoholische Lösungsmittel (D) wenigstens einer ausgewählt aus der Gruppe bestehend aus Ethanol, Propylalkohol und Isopropylalkohol ist.

10. Verfahren nach einem der Ansprüche 1 bis 9, wobei die Zusammensetzung ferner ein schäumendes Paar umfaßt.

11. Verfahren nach Anspruch 10, wobei das schäumende Paar von der Mischung aus einem alkalischen Hydrogencarbonat und einer organischen oder mineralischen Säure gebildet ist, wobei das alkalische Hydrogencarbonat aus Kalziumcarbonat, Natriumcarbonat, Kaliumcarbonat, Natriumhydrogencarbonat, Kaliumhydrogencarbonat sowie den Mischungen dieser Verbindungen ausgewählt ist, und wobei die organische oder mineralische Säure aus Zitronensäure, Fumarsäure, Adipinsäure, Weinsäure, Mononatriumphosphat und Monokaliumphosphat sowie den Mischungen dieser Verbindungen ausgewählt ist.

12. Verfahren nach einem der Ansprüche 1 bis 11, wobei wenigstens ein Trocknungsschritt (d) unter vermindertem Druck bei einem Druck von unter 50 mbar durchgeführt wird.

13. Verfahren nach einem der Ansprüche 1 bis 12, wobei die Schritte (a) bis (d) in einem Reaktor durchgeführt werden, der die folgenden Merkmale aufweist:
- hermetischer Verschluß, der das Aufrechterhalten eines verminderten Druckes ermöglicht, und Mittel zur Sicherstellung eines Teilvakuums,
- Düse(n), die die Zugabe einer Binderlösung ermöglicht (ermöglichen),
- Schwenkwanne, die das Schwingen während der Trocknung ermöglicht,
- Wände mit variabler Temperatur, die derart erhitzt werden können, daß die Trocknung der Körner bewirkt wird,
- eine Schaufel am Boden des Reaktors,
- wenigstens ein Messer mit veränderlicher Länge, das an dem Deckel des Reaktors befestigt ist,
- eine Entleerungsventil für das Herausführen des Produktes.
